# EUROPEAN PATENT APPLICATION

(11) **EP 2 471 768 A1**
(43) Date of publication of application: **04.07.2012**
(21) Application number: 11194216.5
(22) Date of filing: 19.12.2011
(51) Int. Cl.: C07C 209/36

(54) **A method for the catalytic reduction of nitrobenzene to aniline in the liquid phase**

(30) Priority: 29.12.2010 CZ 20100979
(71) Applicant: Vysoká skola chemicko-technologická v Praze, 16628 Praha 6 (CZ)
(72) Inventor: Pasek, Josef, 16900 Praha 6 (CZ); Petrisko, Miroslav, 15200 Praha 5 (CZ)
(74) Representative: Kubickova, Kvetoslava

(57) **Abstract**

The method of the catalytic reduction of nitrobenzene to aniline in the liquid phase, where the hydrogenation is conducted in two reaction zones, where the first zone operates at the temperatures from 150°C to 250°C and the nitrobenzene concentration is kept within the range from 0.05% to 0.5% and the mixture from the first zone is hydrogenated in the second zone at 50°C to 100°C to the residual nitrobenzene concentration of maximally 10 ppm.

## Description

### Field of the Invention

The invention concerns a new production method of aniline by the hydrogenation of nitrobenzene in the liquid phase.

### State of the Art

Aniline is mainly produced by the catalytic reduction of nitrobenzene by hydrogen; in fact, the amination of phenol had been also implemented on an industrial scale but failed to spread later. The nitrobenzene hydrogenation can be conducted through several processes, significantly different one from another. These processes can be divided into two large groups: the gas-phase hydrogenation and the liquid-phase hydrogenation. This invention deals with the liquid-phase hydrogenation with the use of palladium- and platinum-based catalysts or their combinations.

Nowadays, about 85% of nitrobenzene is used for the production of N,N'-diphenyl-methylenediamine (methylenedianiline, MDA), which is the intermediate for the polyurethane materials. The second largest consumer of aniline is the production of the rubber chemicals (10 to 12%), while the rest, accounting for some 2 to 3%, is used in the production of dyes, pharmaceuticals and pesticides.

Palladium and platinum on carriers represent highly-active catalysts for the nitrobenzene hydrogenation. Relating to the metal weight, palladium and platinum are more active by three orders then nickel. With the palladium and platinum catalysts, the nitrobenzene hydrogenation runs at a sufficient rate already at about 30°C and completely selectively. However, any process capable of competing must produce steam utilising the high reaction heat of the nitrobenzene hydrogenation, which is ΔH = - 530 kJ/mol of nitrobenzene and this requires the reaction temperature of at least 150°C or somewhat higher. As the reaction temperature is higher the produced steam pressure may be also higher, which extends the possibilities of the steam utilisation. Nevertheless, above 100°C, Pd and Pt catalyse, to a small extent, the hydrogenation of the aniline aromatic nucleus and the by-product of this reaction - cyclohexylidene imine - is a source of many follow-on products, such as cyclohexylamine, cyclohexanone, cyclohexanol, N-cyclohexylidene aniline, phenylcyclohexylamine, etc. At the same time, it is required that the aniline yield (as per nitrobenzene) should be 99.0% at least. Another problem is caused by a relatively difficult separation of cyclohexanone from aniline.

According to US Patent No. 3 499 034 (priority 1966), aromatic nitro-, dinitro- and nitrochloro derivatives can be hydrogenated in an autoclave at 80 to 200°C and hydrogen pressure of 1.3 to 8.0 MPa under the presence of a Pt or Pd catalyst and the concentration of unreacted nitro-compounds is lower than 0.5%, preferably 0.02% to 0.2%. The product drawn off the autoclave may be used directly for further chemical processing. If the nitro-compound concentration is higher than 0.5%, tarry substances are formed with their intermediate products, azo- and azoxy compounds. In the first place, the process is aimed to the preparation of diamines and the aniline production is not included in the examples. As illustrated by these examples, the nitro-compound concentration in the hydrogenate is even less than 0.01%.

Another patent of E. I. Du Pont de Nemours and company (US 4 185 036, priority 1977) protects the hydrogenation of a mixture of mononitro-compounds and dinitro-compounds or nitro-amino compounds with the use of catalysts based on the VIII group metals at temperatures ranging from 75 to 225°C and pressures from 0.33 to 5.3 MPa. The patent also protects the catalysts containing, in addition to the active metals, iron, nickel or chromium in the range from 0.1% to 10%. If the nitro-amino compound and/or dinitro-compounds are hydrogenated as such, larger quantities of tarry substances are formed - even several percents. The process is aimed to the preparation of diamino-compounds.

According to the Japanese Patent No. 50 157 79, the selectivity of the nitrobenzene hydrogenation is increased by an addition of triethanolamine. In the patent of Mitsui Toatsu Chemicals (EP 0 476 404 A2, priority 1990) it is claimed that during the nitrobenzene (NB) hydrogenation the more by-products are formed by the hydrogenation inside the aniline aromatic nucleus, the less nitrobenzene in the mixture is present. The patent protects the NB hydrogenation process on Pt or Pd catalysts in the temperature range from 150 to 250°C; in this environment, aniline and water formed there evaporate from the reactor and there is, in the liquid phase, less than 0.01% NB; also, a zinc compound is added and the hydrogen stream contains from 1 to 500 ppm of carbon monoxide. Without the addition of CO, the aniline distilling from the reactor contains more than 200 ppm of cyclohexanol, more than 1,000 ppm of cyclohexanone, more than 100 ppm of cyclohexylidene aniline and more than 20 ppm of nitrobenzene. If no zinc salt is dosed into the reactor, even worse results are obtained. Both the zinc salt and CO decrease the extent of the hydrogenation inside the aniline aromatic nucleus. If these additives are combined, the nitrobenzene content in the distillate is less than 10 ppm and the content of all organic impurities is under 100 ppm. It is claimed that the nitrobenzene content in the reaction liquid phase is under 0.01% and according to the equilibrium data the nitrobenzene content in the liquid phase must be max. 20 ppm, if the nitrobenzene content in the distillate is to be kept at 10 ppm.

Another patent of Mitsui Toatsu Chemicals (EP 0458 006 A1, priority 1990) differs in the absence of CO, while the hydrogenation selectivity is being improved by the addition of a zinc compound and alkali metal carbonate or bicarbonate. In examples of both patents, the reaction runs under 0.5 MPa pressure, under which water and aniline can distil off the reaction mixture at a reasonable surplus of hydrogen. However, the process is complicated by the problems connected with the separation of zinc salts and carbonates from the reaction mixture.

The BASF Patent (US 6350911 B1, priority 1998) protects the preparation of amines by the hydrogenation of nitro-compounds in a vertical reactor, where hydrogen is mixed with the liquid in a jet nozzle, into which the liquid circulates from the reactor bottom by a pump. The nozzle is placed in the reactor upper section. Field's Tubes are used in the reactor as cooling elements. In the example, the nitrobenzene hydrogenation is conducted with a Pt catalyst on activated carbon containing some iron (III) hydroxide; at 3 MPa and 180 °C, the yield is 99.5%. The nitrobenzene content in the aniline draw-off stream was maximally 10 ppm, the phenylcyclohexyl amine content maximally 0.1%, with even lower contents of cyclohexanone and cyclohexanol (but not exactly specified). The catalyst content in the reaction mixture was 2%. It seems that - in terms of the hydrogenation inside the aniline aromatic nucleus - platinum is more selective than palladium; however, according to the BASF Patent, even this content of by-products is rather high. In the first place, the process is aimed to the hydrogenation of dinitrotoluenes, where the danger of the nucleus hydrogenation substantially lower.

It stands to reason that the high selectivity of the nitrobenzene hydrogenation to aniline requires a modified platinum catalyst and, if Pd is used, then the nucleus hydrogenation must be blocked by various additives, such as zinc compounds, alkali metal carbonates, CO and/or organic bases.

### Objects of the Invention

The above problems are eliminated by the method of the nitrobenzene hydrogenation to aniline in the liquid phase on Pd- or Pt-based catalysts or their mixture, at 1 to 5 MPa, where the nitrobenzene hydrogenation is conducted in two consecutive reaction zones and where, in the first zone which is very similar to a reactor-mixer, the reaction runs at 150°C to 250°C and the nitrobenzene concentration in the reaction mixture is kept at 0.05% to 0.5% wt. and, having left the first zone, the mixture is further hydrogenated in the second zone similar to the plug flow reactor at 50°C to 100°C down as low as to the residual nitrobenzene concentration of maximally 10 ppm.

More than 99% of nitrobenzene reacts in the first zone at 150°C to 250°C, preferably at 180°C to 210°C. The reaction heat released in the first zone is used for the steam production of 0.3 to 2.0 MPa. In the first zone, the aniline nucleus hydrogenation is curbed by the presence of 0.05% to 0.5%, preferably 0.1 % to 0.3% of nitrobenzene. In the second zone, nitrobenzene completes the reaction with hydrogen on the same suspended catalyst at low temperatures from 50°C to 100°C, i.e. in the range, within which the aniline nucleus hydrogenation runs at an inconsiderable rate. As the case is, the nitrobenzene contained in the reaction mixture leaving the first zone could be separated by an efficient rectification process but it would consume a large amount of heat. It is therefore advantageous that the final reaction mixture contains maximally 10 ppm of nitrobenzene - from such mixture, pure aniline (containing less than 1 ppm of nitrobenzene) can be easily obtained by the rectification.

According to our findings, the nitrobenzene hydrogenation at the nitrobenzene concentration in tenths percents (the first zone) is governed by kinetics nearing to the zero order with respect to the nitrobenzene concentration. As a consequence, the hydrogenation reaction rate in the first zone depends on the nitrobenzene concentration only to a slight extent; however, at very low nitrobenzene concentrations, the hydrogenation is governed by kinetics with the order of 0.7 with respect the nitrobenzene concentration. Therefore, the second reaction zone must be near to the plug flow reactor.

It is therefore expedient that the first zone operates as a reactor-mixer, i.e. so that a medium nitrobenzene concentration in the reactor is maintained in the range of 0.05% to 0.5%. The nitrobenzene presence prevents by its strong sorption on the catalyst the aniline nucleus hydrogenation on the one side, while on the other side, high-molecular substances are formed at higher nitrobenzene concentrations, which could result in the catalyst deactivation. Therefore, a plug flow reactor is not suitable for the first zone. The first zone can be advantageously implemented in the so-called "gas-lift reactor", which is constructed as a circulation loop, in which the circulation of mixture at a rate of 100 to 200 m³ per ton of nitrobenzene feed is supported by hydrogen flow on the principle of mammoth. The circulation principle is based on the different densities in the ascending and descending branches of the loop; the difference is caused by the presence of gas bubbles in the ascending branch. In this simple way, the reaction mixture circulating in the first zone is more than a hundred times higher than the nitrobenzene feed amount and a significant portion of nitrobenzene reacts in the liquid immediately. The circulation at such high rate could be also provided with a centrifugal pump, but it is not convenient and the pump could damage the catalyst or be damaged itself by the catalyst.

The second zone must be maintained in the regime nearing to a plug flow reactor; advantageously, the second zone corresponds to a cascade consisting of four to eight mixers. The mixer cascade can be preferably constructed as a parallel-flow tower with perforated baffles, where the number of baffles is from 5 to 20; alternatively, the second zone could be formed by a parallel-flow cascade of bubbled towers consisting of 2 to 4 members, where each of these towers is fitted with one to three baffles.

In contrast to nickel catalysts, Pd and Pt catalysts may work in a system containing two liquid phases, i.e. aniline and aqueous ones. Nevertheless, even in this case it is advantageous to have a single-phase mixture; therefore, the reaction water is removed by evaporation into the hydrogen surplus that circulates through the first zone. This is sufficient to keep the water concentration in the reaction mixture under 4.5%.

The catalyst content in the reaction mixture must be in the level guaranteeing the sufficient rate of the nitrobenzene hydrogenation in both reaction zones. An exact catalyst composition is not the object of the invention; the Pt-/Pd-based catalysts can be modified appropriately by additions of other metals, such as iron or copper. The catalyst necessary concentration will depend on its activity, i.e. mainly on the concentration of precious metal on a carrier. Catalysts containing from 1% to 5% of precious metals will be suitable. It is important, for the aniline production economy that Pt is approximately 3 times more expensive than Pd, while its activity for the nitrobenzene hydrogenation is 2 to 3 times lower. The necessary catalyst concentration (incl. the carrier) is from 0.05 to 0.3% based on the reaction mixture. Thus, the catalyst concentration is very low and, if sufficient agitation is provided, the catalyst will not clog up the steam generator surface. Once the reaction mixture leaves the second reaction zone, the catalyst is separated by one of generally accepted procedures; a major part of the catalyst will be then recycled into the first zone and only a small part of it is removed from the system and replaced by the fresh catalyst.

To a certain extent, the hydrogenation rate in the first and second zones can be influenced by the reaction temperature and, if necessary, some catalyst may be added into the second reaction zone.

The pressure level used in the process is not critical, but the level from 2 to 3 MPa is favourable because the hydrogen gas produced in the steam reforming of hydrocarbons or the partial oxidation of the heavy oil residue is available exactly at this pressure. Under this operating pressure, the reaction water from the first zone can be removed into the circulating hydrogen gas, the quantity of which is (at the same time) adequate to the gas-lift reactor application, i.e. provides for the necessary circulation of the reaction mixture.

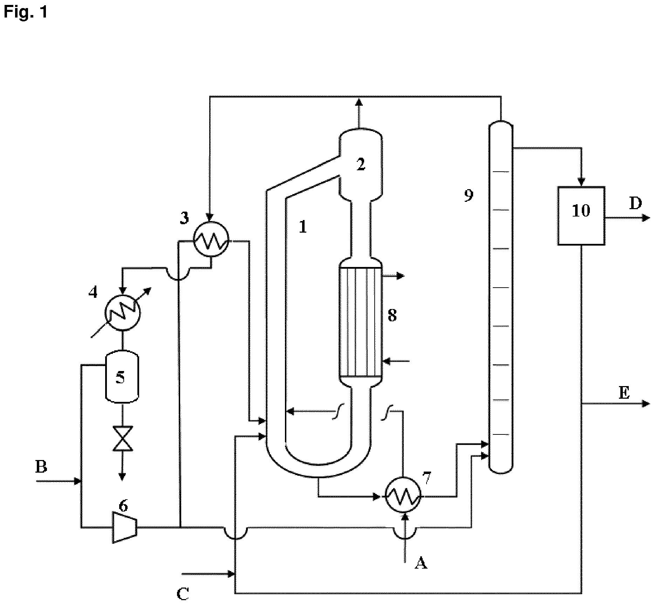

The process option according to this invention with the gas-lift reactor application is demonstrated in Fig. 1 enclosed herewith. The following explanation relates to the figure. From the compressor 6, the hydrogen gas is brought to the gas-lift reactor bottom 1. The stoichiometric hydrogen is added before the compressor from the network (the B Stream). In addition, the suspension (the C Stream) of fresh and recycled catalyst in aniline or water is dosed to the gas-lift reactor bottom. At a slightly higher level, nitrobenzene (the A Stream) is fed into the gas-lift reactor; this stream is heated up by the reaction mixture leaving the first zone, i.e. from the gas-lift reactor, in the heat exchanger 7.

The hydrogen gas causes intensive circulation of the reaction mixture through the lift reactor loop. The circulation rate is 100 - 200 times higher than the quantity of the nitrobenzene feed. Saturated with water and aniline at the reaction temperature of approx. 190°C and 2 MPa pressure, the hydrogen gas is separated in the separator 2, cooled by the heat exchange in the exchanger 3, and further in the cooler 4, to be finally separated from the condensed liquid in the separator 5. After this, the hydrogen is recycled by the compressor 6 to the gas-lift reactor. The necessary liquid circulation rate is achieved at the hydrogen velocity (incl. evaporated substances) of 0.5 to 1.0 m per second as per the whole cross section of the lift reactor. The condensed liquid is formed by the aniline layer and the aniline water layer and it is separated in the phase separator. The aniline layer contains some nitrobenzene and it is added into the second reaction zone.

On the descending branch of the gas-lift reactor loop, the steam generator 8 is placed; 6-bar steam is produced here and the reaction mixture is cooled down by 10 to 13°C.

A part of the circulating mixture containing approx. 0.2% of nitrobenzene is cooled from 175 to 180°C down to 80°C by the heat exchange with nitrobenzene in the heat exchanger 7 and then it is brought into the reactor bottom 9 of the second reaction zone. A relatively small portion of hydrogen is also added into the reactor bottom from the compressor 6. The second reaction zone consists of the bubbled tower 9 with eight perforated baffles that restrict the axial agitation. In the second reaction zone at 80°C and 2 MPa, nitrobenzene continues to react down to the concentration of approx. 5 ppm. The catalyst is separated from the reaction mixture by one of normally used processes 10 and a major part off the separated catalyst is returned into the first reaction zone or, as applicable, to the reactor bottom of the second reaction zone. Raw aniline (the D Stream) free of the catalyst is then purified by a normal rectification process in a series of columns. A part of the separated catalyst is brought out of the system (the E Stream) to be replaced with the fresh catalyst (the C Stream).

### Example of the Invention

Into a continuous agitated autoclave with the liquid hold up of 500 ml and total volume of 1 litre the following feed was fed during one hour:
200 g of nitrobenzene,
0.5 g of catalyst (3 g Pd on activated carbon containing 3% Fe₂O₃) as a suspension in aniline,
15 mol of hydrogen gas at pressure 2 MPa

In the autoclave that represented the first reaction zone the temperature level of 150°C was maintained by cooling. The hydrogen surplus leaving the autoclave was cooled and the condensed liquid was separated into the aqueous and aniline phases. In the autoclave, the nitrobenzene concentration was kept in the range from 0.1 % to 0.3% by the quantity of the fed catalyst.

While keeping the same level in the autoclave, the reaction mixture was drawn off to the second reaction zone, which consisted of a tube with 20 mm internal diameter and 3-metre height and fitted with six perforated baffles. Into the bottom of the second reaction zone, 0.5 mol/hour of hydrogen gas was brought at 2 MPa.

The reaction mixture was leaving from the top of the tube and - after filtration - the raw aniline product contained 3% of water, 5 ppm of nitrobenzene and 200 ppm of aniline nucleus hydrogenation products. After the raw product rectification, the final aniline product contained less than 1 ppm of nitrobenzene, 5 ppm of cyclohexylamine, 30 ppm of cyclohexanol and 5 ppm of phenol.

### Industrial applicability

This Invention is applicable in the chemische industry for prepare aniline

## Claims

1. The method of the nitrobenzene hydrogenation to aniline in the liquid phase on Pd- or Pt-based catalysts, or their mixture, and at 1 to 5 MPa **characterised in that** the nitrobenzene hydrogenation is conducted in two consecutive reaction zones and where, in the first zone, which is very similar to a reactor-mixer hydrogenation runs at 150°C to 250°C and the nitrobenzene concentration in the reaction mixture is kept at 0.05% to 0.5% wt. and the mixture from the first zone is further hydrogenated in the second zone very similar to the plug flow reactor at 50°C to 100°C down as low as to the residual nitrobenzene concentration of maximum 10 ppm.

2. The method according to Claim 1 **characterised in that** in the first reaction zone, the reaction heat of the nitrobenzene hydrogenation is removed to boiling water producing thus steam within the pressure range from 0.3 to 2.0 MPa.

3. The method according to Claim 1 and Claim 2 **characterised in that** in the first reaction zone, the mixture is circulated at the rate of 100 to 200 m³ per ton of the nitrobenzene fed into the circulation loop and where the circulation is supported by the hydrogen flow on the principle of a mammoth pump.

4. The method according to Claim 1 and Claim 2 **characterised in that** in the first reaction zone the hydrogenation is conducted in the bubbled tower with internal circulation.

5. The method according to Claim 1 and Claim 2 **characterised in that** in the second reaction zone the hydrogenation is conducted in the parallel-flow tower with perforated baffles, where the tower height/diameter ratio is from 50 to 150 and the number of baffles from 5 to 20.

6. The method according to Claim 1 and Claim 2 **characterised in that** in the second reaction zone, the hydrogenation is conducted in the parallel-flow cascade of bubbled towers consisting of 2 to 4 members, where each of these towers is fitted with one to three baffles.
